# EUROPEAN PATENT APPLICATION

(11) **EP 4 273 222 A1**
(43) Date of publication of application: **08.11.2023**
(21) Application number: 21914753.5
(22) Date of filing: 29.12.2021
(51) Int. Cl.: C12N 1/20, A23L 3/3571, A23C 9/123, A23C 19/16, B65D 81/28, C12R 1/25

(54) **LACTOBACILLUS PLANTARUM STRAIN, USE AS A PROBIOTIC AND BIOACTIVE PRODUCT DERIVED THEREFROM**

(30) Priority: 30.12.2020 ES 202031317
(71) Applicant: Universitat de Valéncia, 46010 Valencia (ES); AIMPLAS - Asociación de Investigación de Materiales Plásticos y Conexas, 46980 Paterna (ES)
(72) Inventor: MECA DE CARO, Giuseppe, 46010 Valencia (ES); MAÑES VINUESA, Jordi, 46010 Valencia (ES); LUZ MINGUEZ, Carlos, 46010 Valencia (ES); RODRIGUEZ GARRIDO, Lorena, 46980 Paterna (Valencia) (ES); SANTOMÉ ZUAZUA, Jezabel, 46980 Paterna (Valencia) (ES); GOMEZ JIMENEZ, Lola, 46980 Paterna (Valencia) (ES)
(74) Representative: Temiño Ceniceros, Ignacio
(86) International application number: PCT/ES2021/070943
(87) International publication number: WO 2022/144483

(57) **Abstract**

The invention relates to a *L. plantarum* strain, probiotic use of the same by means of a gastro-resistant capsule, and a bioactive product coated with an antimicrobial whey coating formulation obtained by fermenting whey using said strain. It also relates to a method for obtaining the same and food use of the bioactive product, preferably to inhibit fungal growth in cheese.

## Description

### FIELD OF THE INVENTION

The invention relates to a *Lactobacillus plantarum* strain and the use thereof as a probiotic and for obtaining a formulation comprising fermented whey. This formulation is applied to a food film, separator or protector, generating a bioactive product that prevents the growth of microorganisms during storage of the food in question and that prolongs its shelf life.

### BACKGROUND

According to the latest indications of the European Food Safety Authority (EFSA), a large part of degenerative diseases arise from food, and that is why there is a real demand to reduce the use of additives and use less aggressive food processing techniques, with the aim of producing minimally processed foods that meet the organoleptic characteristics provided by fresh or traditional food, in addition to beneficial probiotics for displacing microorganisms harmful to health and aiding digestion.

Although it is proven that the content of toxins and pathogenic microorganisms present in food can be reduced by using several physical and biological techniques, such as heat treatments and fermentations by bacteria and yeast, in the scientific literature there are few studies that evaluate reducing the content of toxins or mycotoxins in food by using natural active ingredients, despite the potential health benefit for consumers that could be obtained.

Due to all this, the food industry, pressured by market demands, undergoes the development of new products and food transformation processes to obtain safe products that have the characteristics desired by consumers. One clear example of development occurs in the food fermentation industry, where starter cultures play a very important role in preparing the final product, contributing to the food safety and organoleptic quality thereof.

Probiotics are organisms, generally bacteria, that are considered beneficial rather than detrimental to their animal host. Interest in probiotics waned with the advent of antibiotics. However, with the emergence of antibiotic-resistant bacteria, there is a renewed interest in probiotic bacteria, which are now defined as "live microorganisms that, when administered in adequate amounts, confer a health benefit to the host". It is now a popular concept that the accumulation of probiotic organisms in the intestine is beneficial to the overall health of the host organism and there are reports that probiotic administration is useful in maintaining dietary health and proper digestion.

The document Use of whey fermented by lactic acid bacteria as a natural preservative with antifungal properties. in Annals of Nutrition and Metabolism (http://semipyp.es/pdf/workshop/workshop9_2018_abstracts.pdf) demonstrates the antimicrobial characteristics of whey fermented by lactic acid bacteria, but no reference is made to any practical application; the present invention relates to a specific strain, the strain CECT30089 that exhibits a minimum inhibitory concentration and a minimum fungicidal concentration twice as low as those of the document.

The scientific document by Garcia Ruiz et al, (2014) Evaluación de las propiedades probióticas de bacterias lácticas de origen enológico. Alim. Nutri. Salud, Vol. 21, No. 2, pp. 28-34, 2014 discloses the probiotic properties of a series of lactic bacteria; however, the *L. plantarum* strain of the present invention exhibits better capacities for adhesion to intestinal cells and displacement of pathogenic microorganisms than the disclosed strains.

Meetings these demands entails a great challenge for the food industry, mainly when the desired characteristics entail the elimination of synthetic preservatives in order to obtain more natural products with greater antimicrobial activity. As a result, biopreservation has attracted the attention of the food industry as a potential replacement for preservatives used today.

In recent years, biopreservation has been positioned as a substitute for the preservatives currently used, as well as for antimicrobial compounds and absorbent solutions, since they have the advantage of using bacteriocins produced by bacteria considered beneficial for health in food preservation. It has been reported that these purified or semi-purified compounds can be used as biopreservatives in foods in order to reduce or eliminate certain spoilage microorganisms and some pathogens.

The antimicrobial solutions discussed above have certain limitations in some cases, which explains the low inclusion of these solutions on the market:
- they give smell and flavour to the food: essential oils, cinnamaldehyde.
- they are not approved solutions for food contact because they are not included on the list of substances admitted for this use, in accordance with regulation 10/2011 on plastic materials and articles intended to come into contact with food and regulation 450/2009 on active and intelligent materials and articles intended to come into contact with food, this is the case of sorbic acid and lauric alginate ester.
- they do not have the necessary effectiveness compared to the synthetic additives used in preparing a packaged food product.

Active packaging is a field of application that is currently under development. This packaging can be considered as packaging in which secondary components (active agents or elements) have been deliberately included to improve the food preservation conditions and prolong its shelf life. The packaging actively participates in preserving the product and maintaining or enhancing its organoleptic properties, either by absorbing compounds that deteriorate the product or by emitting substances that help to preserve it. We are faced with numerous related lines of work: packaging with oxygen and moisture absorbers, and packaging with additives having antimicrobial agents and antioxidants. Each type of active packaging is useful for one or more types of food product.

Active packaging, unlike traditional packaging that must be totally inert, is designed to actively and continuously interact with its content, its purpose being to extend the preservation time or maintain or improve the condition of the packaged food. Its use is increasingly widespread, especially in fruit, vegetables, fish and meat.

Among the different active packaging technologies that exist, in the packaging of commercial food products the most widespread technology is the oxygen absorber, which contains chemicals that eliminate residual oxygen from the atmosphere surrounding the cheese. Exposure to oxygen can cause, among others, the growth of mould and aerobic bacteria, as well as changes in the nutritional composition or physiological changes such as the respiration rate.

Regarding packaging for food use, in the specific case of cheese for food use, in the specific case of cheese, a separator or film is usually used to facilitate its handling, which can be a film made of paper or polymer material, a non-stick material that allows the end consumer to take a single slice without it sticking to another due to the action of fat or moisture.

Patent documents CN101747635A, WO2000049899A1 and CN103351471A describe monolayer films for food use that exclusively comprise whey proteins such as casein. However, unlike previous documents, the bioactive product of the present invention is coated with the antimicrobial whey coating formulation obtained from the fermentation product of a lactic acid bacteria strain that also contains proteins such as casein, a biocomplex comprising other acids, peptides and bacteriocins, responsible for the antifungal effect. Therefore, the present invention has two advantages with respect to said documents: the use of industry waste that is whey (circular economy) and a greater capacity for fungal inhibition due to the greater variety of compounds.

The scientific article, Karimi et al., 2020, *Preparation and characterization of whey protein isolate*/*polydextrose-based nanocomposite film incorporated with cellulose nanofiber and L. plantarum: A new probiotic active packaging system,* discloses a biodegradable film made with polydextrose that is coated with whey and non-whey proteins fermented with lactic acid bacteria. In the present invention, the active packaging material is a non-biodegradable plastic that incorporates an antimicrobial whey coating formulation comprising whey fermented by lactic acid bacteria. Furthermore, both films have a different purpose; in the scientific article the antibacterial activity of the film has been tested on pathogenic microorganisms of the gastrointestinal tract, while the film of the present invention has been tested against fungal pathogens.

The present invention relates to a *L. plantarum* strain, its use as a probiotic by means of a gastro-resistant capsule, as well as a method for producing a bioactive product with an antifungal coating formulation, the intermediate products of said method and the uses thereof. The invention has the advantage of not having to resort to synthetic antimicrobial additives, which can potentially cause different problems to consumers' health in the long term.

### DESCRIPTION

In the present specification, the term "lactic acid bacteria" (LAB) refers to food grade bacteria that produce lactic acid as the main metabolic end product of carbohydrate fermentation. These bacteria are related by common metabolic and physiological characteristics and, in general, are gram-positive bacilli or cocci, with low guanine-cytosine content, acid-tolerant, non-sporulating, and rod-shaped. During the fermentation stage, the consumption of sugars by these bacteria causes the formation of lactic acid and lowers pH. Thus, these bacteria are responsible for acidification and, in some cases (for example, in the case of milk fermentations), for the texture of the fermented product. This term encompasses the bacteria of the strain of the invention, the strain CECT30089.

The term "milk" is intended to encompass milk from mammals, milk from plant sources or recombinantly produced milk. Preferably, the milk is from a mammalian source. Sources of milk from mammals include, among others, cows, sheep, goats, buffaloes, camels, Ilamas, mares and deer. In one embodiment, the milk is from a mammal selected from the group consisting of cow, sheep, goat, buffalo, camel, Ilama, mare and deer, and a milk mixture of combinations thereof. Sources of milk from plants include, among others, milk extracted from soya bean, pea, peanut, barley, rice, oat, quinoa, almond, cashew, coconut, hazelnut, hemp, sesame seed and sunflower seed and combinations thereof. Furthermore, the term "milk" refers not only to whole milk, but also to skimmed milk or any liquid component derived from same.

The term "fermentation" as used herein refers to a metabolic process in which sugars are converted to acids, gases or alcohol. Preferably, fermentation comprises the conversion of lactose to lactic acid.

The term "starter culture" as used herein refers to a composition comprising one or more lactic acid bacteria, which are responsible for the acidification of the fermentation substrate. Compositions of the starter cultures can be fresh (liquid), frozen or lyophilised. Lyophilised cultures must be activated before use.

The terms "starter culture" and "inoculum" are synonymous and are used interchangeably.

The term "biocomplex" is the set of molecules produced by microorganisms during whey fermentation with antimicrobial activity.

In the present invention, the strain "CECT30089" and "BN17" are interchangeable terms and are used interchangeably.

The term "antimicrobial" as used herein comprises the terms "antibacterial and/or antifungal".

In the present invention, the terms "tablets" and "capsules" are interchangeable terms and are used interchangeably.

The present invention relates to a *Lactobacillus plantarum* strain deposited in the Spanish Type Culture Collection with number 30089 or mutants derived from same, wherein said mutants have the same or better antimicrobial properties than the strain CECT30089.

This strain was deposited on 26 February 2020 in the Spanish Type Culture Collection, Calle Catedrático Agustín Escardino, 9, Postal Code 46980, Paterna (Valencia), Spain in accordance with the provisions of the Budapest Treaty.

The mutants derived from this strain relate to strains that are derived from CECT30089, or obtained from CECT30089, and may have mutations compared to *Lactobacillus plantarum;* preferably, the mutant strain has the same or better antifungal properties than the parent strain CECT30089. Preferably, the mutant derived from CECT30089 has at least 80% or more, preferably at least 90% or more, more preferably at least 95% or more, or even up to 100% or more than 100% of the antimicrobial effect as compared to the strain CECT30089 under the same conditions.

The antimicrobial properties are selected from antifungal properties, antibacterial properties and combinations thereof.

Another aspect of the invention relates to the use of the *Lactobacillus plantarum* strain defined above, as an antimicrobial growth inhibitor in food products.

Another additional aspect of the invention relates to the probiotic use of the *Lactobacillus plantarum* strain defined above.

Another additional aspect of the invention relates to a gastro-resistant capsule for use as a probiotic that comprises the strain of bacteria defined above.

In a particular embodiment, the gastro-resistant capsule comprises milk from mammals, whey from mammals, plant milk, plant whey and combinations thereof.

The milk is selected from skimmed milk, semi-skimmed milk, whole milk and combinations thereof.

The gastro-resistant capsule may comprise at least one additive, for example, flavourings such as sucrose, gelatine, cellulose, starches, excipients, such as maltodextrin, bovine serum albumin, binders, anti-caking agents, preservatives and other pharmaceutically acceptable delivery carriers or ingredients to enhance taste, palatability or other organoleptic properties.

These additives can be evenly distributed throughout the capsule or concentrated in at least one specific portion of the same.

The bacteria of the strain CECT30089 may be the only probiotic ingredient, or the main probiotic ingredient in the gastro-resistant capsule.

In a particular embodiment, the gastro-resistant capsule comprises skimmed milk, for example, ultrapasteurised skimmed milk.

Skimmed milk is a fraction of whole milk, which is obtained when the fat (cream) is removed from the milk. The fat in milk is present in whole milk in the form of globules, which are composed mainly of triacylglycerols and are surrounded by a membrane consisting of complex lipids such as phospholipids, along with proteins. The lipids in milk are 97-98% triacylglycerols, small amounts of di- and monoacylglycerols, free cholesterol and cholesterol esters, free fatty acids and phospholipids. Whole milk from cows comprises approximately 4% milk fat. Commercially available skimmed milk may still have a low milk fat content of up to 0.3%. According to the invention, the fat content of skimmed milk is, for example, less than 0.5%, or it may be less than 0.3% or less than 0.1 %, or even about 0% by weight.

In another additional embodiment, the milk is semi-skimmed milk. Semi-skimmed milk may comprise between approximately 0.5 and approximately 2.5% by weight of milk fat.

In another additional embodiment, the milk is whole milk. This milk contains all its components. Whole milk can be used without any heat treatment. However, whole milk can also be used after pasteurisation and/or homogenisation. Alternatively, dry whole milk or powdered whole milk can be used. Dried whole milk or powdered whole milk are usually obtained by removing water from pasteurised and homogenised whole milk. In this embodiment, the gastro-resistant capsule may comprise between approximately 2.5 and approximately 6%, for example, between approximately 3 and 5% by weight of milk fat. Semi-skimmed milk and whole milk comprise skimmed milk as a fraction of the same. In general, the capsule may comprise between 0 and 6% by weight of milk fat. Milk can be UHT milk (ultrapasteurised milk). Skimmed milk, semi-skimmed milk or whole milk can be provided as a powder that is redissolved in the composition. Skimmed milk can be diluted, preferably with water or a buffer. In this embodiment, the capsule may comprise from 10 to 95%, for example, from 20 to 80% of skimmed milk.

Milk can be of plant origin, the concentrations of fat in plant milk may be lower than those in animal milk.

The concentration of bacteria in the gastro-resistant capsule for probiotic use is comprised in the range between 5% and 50% by weight, more preferably between 10% and 40%, even more preferably 15% and 30%.

The number of bacteria in the gastro-resistant capsule is between 10⁴ and 10¹⁵, preferably between 10⁶ and 10¹³, more preferably between 10⁸ and 10¹² cells, more preferably between 10⁹ and 10¹¹ cells. The number of cells can be determined by known methods, for example, with a cell counter.

Probiotic use is preferably gastrointestinal, and can be used in humans and/or animals, preferably humans.

The probiotic effect of the strain CECT30089 comprises an antimicrobial effect, preferably an antifungal and/or antibacterial effect.

In a particular embodiment, the affected microorganisms are pathogenic microorganisms, for example, *Candida* spp., *Shigella dysenteriae, Listeria monocytogenes, Salmonella enterica, Escherichia coli, Clostridium perfringens, Staphylococcus aureus,* and/or *Yersinia enterocolitica.*

A method of use that comprises administering a composition comprising bacteria of the strain CECT30089 and a gastro-resistant capsule to a subject.

Another additional aspect of the invention relates to an antimicrobial formulation that comprises the fermentation product of a substrate by the strain described above from which the bacteria have been separated.

Some examples of antimicrobial bioactive substances contained in the antimicrobial formulation are organic and phenolic acids, bioactive peptides, bacteriocins, casein, and biocomplexes of molecules responsible for antifungal and antibacterial activity: phenylactic acid (PLA), in concentrations greater than 200 mg/l, and of another 19 different compounds therebetween, ferulic acid, caffeic acid, syringic acid, benzoic acid, gallic acid, etc., with variable concentrations between 10 and 250 mg/l.

The fermentation substrate can be milk, whey and/or combinations thereof in any ratio, preferably whey.

The fermentation substrate is selected from milk from mammals, whey from mammals, plant milk, plant whey and combinations thereof. Milk or whey from mammals may preferably come from cows, sheep, goats, buffaloes, camels, Ilamas, mares, deer and milk mixtures thereof, more preferably cows, sheep, goats and combinations thereof. Plant milk or whey can come from soya bean, pea, peanut, barley, rice, oat, quinoa, almond, cashew, coconut, hazelnut, hemp, sesame seed, sunflower seed and combinations thereof.

In a particular embodiment, whey from cow's milk, sheep's milk, goat's milk or combinations thereof is used.

In a preferred embodiment, whey from goat's milk is used.

Whey can be sweet whey or acid whey, the first being obtained by enzymatic coagulation of milk caseins and the second by acid coagulation. In a particular embodiment, sweet whey is used.

Whey is a waste material from the dairy industry and is obtained following a method known in the state of the art.

Another additional aspect of the invention relates to a method for producing the previously described antimicrobial formulation that comprises the following steps:
a. Fermenting the substrate with a starter culture comprising at least one strain of lactic acid bacteria described above and
b. at the end of fermentation, separating the lactic acid bacteria from step a) by centrifugation.

The fermentation substrate is selected from milk, whey and combinations thereof in any ratio, preferably whey.

The antimicrobial formulation has an antifungal and/or antibacterial effect.

The substrate can be fermented at a temperature comprised between 4 and 50°C, preferably between 10 and 49°C, more preferably between 15 and 48°C, even more preferably between 20 and 47°C, and even more preferably between 30 and 45°C for a time interval comprised between 20 and 96 hours.

In a particular embodiment, the substrate can be previously pasteurised to eliminate the existing microorganisms that could interfere with the subsequent fermentation process. Pasteurisation can be performed following the standard conditions of the technical sector.

The starter culture can be activated prior to fermentation by incubating it in the same substrate as the one wherein the fermentation is to be carried out later, using standard time and temperature conditions in the area. After activation of the starter culture, scaling to the substrate that will be fermented can be carried out in a ratio comprised in the range between 1:80 and 1:120 of volume of the initial culture:volume of the substrate to be fermented.

The initial concentration of the starter culture in step a) of the method is from 10⁴ to 10¹² CFU/ml (Colony Forming Units per ml) of the inoculum, preferably between 10⁸ and 10¹⁰ CFU/ml of the inoculum.

At the end of fermentation, centrifugation is carried out to eliminate the bacteria from the fermented whey.

In another particular embodiment, the antimicrobial formulation can be lyophilised after the bacteria separation step. Lyophilisation can be performed by following any state-of-the-art protocol.

Another additional aspect of the invention relates to a method for obtaining a bioactive product that comprises the following steps:
a) preparing a solution of the antimicrobial formulation
b) dissolving at least one polymeric matrix in water
c) mixing the product of step a) with the product of step b) to obtain an antimicrobial whey coating formulation and
d) applying the formulation obtained in step c) on a material, to obtain a bioactive product.

Prior to obtaining the coating formulation, the antimicrobial formulation was dissolved in water, (step a) by means of magnetic stirring for a time interval between 12 and 36 hours until complete dissolution. The dissolution conditions of the antimicrobial formulation can vary to favour its complete dissolution: it can be used with heat, up to a maximum of 40°C, and stirring, up to a maximum of 800 rpm (revolutions per minute). A person skilled in the art would know how to choose the ideal conditions for this step.

The solutions of step a) can have a concentration of between 1 % and 40% of antimicrobial formulation in water, preferably between 5% and 30%, even more preferably between 10% and 35%. The concentration depends on the solubility of whey. A higher or lower solubility can be obtained based on the starting substrate. The higher the protein content, the less soluble it will be. The higher the concentration of the antimicrobial formulation in water, the higher its viscosity will be.

In a particular embodiment, the concentration of the antimicrobial formulation is between 20% and 25%.

The polymeric matrix of step b) must be a polymeric matrix suitable for food contact, preferably polyvinyl alcohol (PVOH), Ethylene-Vinyl-Alcohol (EVOH) copolymers, styrene-acrylic copolymers, acrylic resins, anionic aqueous dispersions, aqueous dispersions, aliphatic polyurethane resins, acrylic-urethane copolymers, polyurethanes, alkyd resins, PVC, polyvinyl acetate, nitrocellulose, polyvinyl butyrate (PVB), polyester (PET), polyester acrylates, urethanes, chitosan, alginates, Hydroxypropyl methylcellulose (HPMC), starch, aminoplast polyethylene glycol solutions, polysaccharides, fatty acids, proteins, waxes, rubbers, collagen, pectins, gelatines and composites.

The solutions of step b) can have a concentration of 0.01 % to 40% of the polymeric matrix in water, preferably between 0.5% and 35%, more preferably between 1% and 30%, even more preferably between 2% and 25%, and even more preferably between 5% and 20%.

As any person skilled in the art will recognise, a more or less high concentration can be reached based on the type of polymeric matrix. For example, in the case of alginate or chitosan, a concentration in water of 2% is already a very high concentration; in contrast, polymeric matrices from fossil sources or acrylic resins allow for a higher concentration.

Concentration is closely related to the viscosity of the matrix; in the present application, the viscosity has been measured with a Ford #4 cup.

Steps a) and b) can be performed simultaneously or in any order.

The product of step c) can have a concentration of 1 % to 25% of the product of step a) in b).

The antimicrobial formulation is incorporated into the dissolved polymeric matrix at a temperature between 20 and 50°C, since in the case of raising the temperature excessively the antimicrobial whey coating formulation may lose antimicrobial properties. The incorporation of the antimicrobial formulation is carried out at stirring between 500-1000 rpm. Vigorous stirring can produce foam that makes it difficult to apply later. To ensure homogeneity of the antimicrobial coating, stirring is maintained between 1 and 12 hours. Subsequently, the solution is acidified until the pH is in the range between 2 and 5; any acid suitable for food use can be used, preferably a weak acid, more preferably lactic acid. This acid can be found in the starting substrate.

The adjustment of pH to these values is important for the activity of the antimicrobial molecules of the biocomplex.

In a particular embodiment, steps a) and c) can be performed simultaneously, in other words, adding the antimicrobial formulation to the dissolved polymeric matrix.

In another particular embodiment, pH must be regulated to a range between 2 and 5, preferably 3, or to the pH that the antimicrobial formulation had after fermentation with the strain CECT30089.

In a particular embodiment, pH is regulated by adding an acid, for example, acetic acid, lactic acid, malic acid, fumaric acid, citric acid, succinic acid, ascorbic acid, formic acid, phosphorus acid, sorbic acid, tartaric acid, adipic acid, and/or nicotinic acid.

In another particular embodiment, step c) comprises the incorporation of the product of step a), in other words, the solution of the antimicrobial formulation to a polymeric matrix in an aqueous solution, into the product of step b).

In another particular embodiment, step c) comprises the incorporation of the product of step b) into the product of step a).

In another particular embodiment, steps a), b) and c) can be performed simultaneously.

Optionally, at least one necessary additive can be added to ensure correct subsequent application on the selected material. Some of the additives include:
- surfactants, for example, silicone-free hydrophobic solids, polysiloxanes and polysiloxane aqueous solutions, polyether-modified polydimethylsiloxane, paraffin-based oils, polysiloxanes in polyglycol, alkylammonium salts and polymethylalkylsiloxane.
- rheological additives, modified urea solutions, modified hydrophobic polyurethane solution, polyurethanes, aminoplast polyethylene glycol solution, castor oil derivatives, modified urea solutions, polyhydroxycarboxylic acid amide solutions, polyurethane solutions with urea, urea-modified medium-polarity polyamides, polycarboxylic acid ester, and functional oligoamide amine solution,
- food acids such as acetic acid, lactic acid, malic acid, fumaric acid, citric acid, succinic acid, ascorbic acid, formic acid, sorbic acid, tartaric acid, adipic acid, nicotinic acid and phosphoric acid, preferably phosphoric acid.

The surfactants and adhesion promoters used depend on the polymeric matrix used, a person skilled in the art would know what specific combination of additives with polymeric matrices can be used.

The added additives will not exceed 15% of the total volume of the antimicrobial whey coating formulation, preferably the percentage will not be greater than 10% of the total volume of the antimicrobial whey coating formulation.

In step d) the antimicrobial whey coating formulation as a coating on the material to obtain a bioactive product can be applied with printing equipment or an application of coatings on a laboratory scale or on an industrial scale using conventional printing technologies, such as flexography or rotogravure or spraying.

The use of any of the technologies indicated in the previous paragraph is carried out following the instructions of the equipment manufacturer or any protocol known in the technical sector.

The material on which the coating formulation is applied is selected from plastic, preferably PE (polyethylene), polypropylene (OPP), polyethylene terephthalate (PET), low-density polyethylene (LDPE), high-density polyethylene (HDPE), Ethylene-Vinyl-Alcohol (EVOH), paper and ethylene vinyl acetate (EVA), corn starch, polypropylene (PP), polypropylene modified by photoinitiated polymerisation of acrylic acid (PP-g-PAA), polylactic acid (PLA), polyvinyl chloride (PVC), chitosan, fish gelatine, cellulose, paper and combinations thereof.

This material can be any product or packaging suitable for introducing food therein, preferably a film and/or a separator.

Once the antimicrobial whey coating formulation has been applied on the material, the bioactive product is obtained.

Bacteria are eliminated at the end of the fermentation process, whereby neither the antimicrobial formulation, the antimicrobial whey coating formulation nor the final bioactive product contain microorganisms.

In a particular embodiment, an adhesion promoter primer solution may be applied on the material prior to applying the antimicrobial whey coating formulation. The advantage of applying this first coating layer is an improvement on the adhesion capacity of the coating formulation on the material, and therefore it allows less antimicrobial formulation to be used in the antimicrobial whey coating formulation for the second coating application, thus reducing the costs of the method.

The primer solution can be the same or a different polymeric matrix used in the antimicrobial whey coating formulation, for example, polyvinyl alcohol (PVOH), Ethylene-Vinyl-Alcohol (EVOH) copolymers, styrene-acrylic copolymers, acrylic resins, anionic aqueous dispersions, aqueous dispersions, aliphatic polyurethane resins, acrylic-urethane copolymers, polyurethanes, alkyd resins, PVC, polyvinyl acetate, nitrocellulose, polyvinyl butyrate (PVB), polyester (PET), polyester acrylates, urethanes, chitosan, alginates, Hydroxypropyl methylcellulose (HPMC), starch, aminoplast polyethylene glycol solutions, polysaccharides, fatty acids, proteins, waxes, rubbers, collagen, pectins, gelatines and composites.

It must be dissolved in water or any other solvent approved for food contact and can have a concentration of 0.01 % to 40% of the coating solution or primer in the solvent, preferably between 0.5% and 35%, more preferably between 1% and 30%, even more preferably between 2% and 25%, and even more preferably between 5% and 20%.
and its application on the material can be through conventional printing technologies, such as flexography or rotogravure or spraying.

An additional object of the invention relates to an antimicrobial whey coating formulation obtained from lactic ferment of the strain CECT30089 that comprises a polymeric matrix dissolved in water. In other words, the intermediate product of step c) of the method for obtaining a bioactive product.

The polymeric matrix is selected from polyvinyl alcohol (PVOH), Ethylene-Vinyl-Alcohol (EVOH) copolymers, styrene-acrylic copolymers, acrylic resins, anionic aqueous dispersions, aqueous dispersions, aliphatic polyurethane resins, acrylic-urethane copolymers, polyurethanes, alkyd resins, PVC, polyvinyl acetate, nitrocellulose, polyvinyl butyrate (PVB), polyester (PET), polyester acrylates, urethanes, chitosan, alginates, Hydroxypropyl methylcellulose (HPMC), starch, aminoplast polyethylene glycol solutions, polysaccharides, fatty acids, proteins, waxes, rubbers, collagen, pectins, gelatines and composites.

Another additional object of the invention relates to a bioactive product obtained by the method described above that comprises an antimicrobial whey coating formulation and a material.

The material is selected from plastic, preferably PE (polyethylene), polypropylene (OPP), polyethylene terephthalate (PET), low-density polyethylene (LDPE), high-density polyethylene (HDPE), Ethylene-Vinyl-Alcohol (EVOH), paper and ethylene vinyl acetate (EVA), corn starch, polypropylene (PP), polypropylene modified by photoinitiated polymerisation of acrylic acid (PP-g-PAA), polylactic acid (PLA), polyvinyl chloride (PVC), chitosan, fish gelatine, cellulose, paper and combinations thereof.

The bioactive product can be bioactive packaging, a bioactive film and/or a bioactive separator. It can be any container as long as it is coated with the antimicrobial whey coating formulation.

In an additional embodiment, the bioactive product can be in the form of a sheet, a film and/or packaging, preferably a film.

Another additional aspect of the invention relates to the use of the bioactive product as an antimicrobial growth inhibitor in food products.

In a particular embodiment, the bioactive product can be used as a fungal growth inhibitor in food products.

The bioactive product must be in direct contact with the food product.

The food products can be dairy products and preferably cheese.

In a particular embodiment, the bioactive product inhibits the growth of fungal species selected from among *P. camemberti, P. expansum, P. roqueforti, P. brevicompactum, P. commune, P. verrucosum* and combinations thereof.

Unless otherwise stated, all the technical and scientific terms used herein have the same meaning that is commonly understood by a person with ordinary skill in the art to which this invention belongs. Methods and materials similar or equivalent to those described herein may be used to implement the present invention. Throughout the description and the claims, the word "comprises" and its variations are not intended to exclude other technical features, additives, components or steps. The objects, advantages and additional features of the invention will become apparent to those skilled in the art upon examination of the description or may be learned by implementing the invention. The following examples and drawings are provided by way of illustration and do not seek to limit the present invention.

### Brief description of the figures

**Figure 1** shows a count of *Lactobacillus plantarum* BN17 during gastrointestinal digestion. Hours 1 and 2 simulate the conditions of stomach digestion, hours 3-5 simulate conditions in the small intestine and 24 hrs simulate digestion in the colon, large intestine. The standard error (SEM) is given for each condition.
**Figure 2** shows the feasibility of *Lactobacillus plantarum* BN17 in a gastro-resistant capsule for 21 days stored at 20°C.
**Figure 3** shows a count of *Lactobacillus plantarum* BN17 encapsulated and lyophilised during gastrointestinal digestion. Hours 1 and 2 simulate the conditions of stomach digestion, hours 3-5 simulate conditions in the small intestine and 24 hrs simulate digestion in the colon, large intestine. The standard error (SEM) is given for each condition.
**Figure 4** shows SDS-PAGE electrophoresis of proteins of whey fermented by lactic acid bacteria, LAB. M=High molecular weight marker, m=low molecular weight marker, a= control, b= strain BN16, c= strain E4, d= strain BN17. α-LA= Alpha-lactalbumin, β-LG=Beta-lactoglobulin.
**Figure 5** shows a representative image of the inhibitory effect of the strain BN17 on *Penicillium spp.* (5 species) in a culture dish.
**Figure 6** shows a print on PET of the antimicrobial whey coating formulation PRO19-0073-01-01.
**Figure 7** shows an activity assay of the bioactive product in sliced mozzarella cheese. (A) Control cheese contaminated with *P. commune* (B) Cheese contaminated with *P. commune* and treated with a bioactive film.
**Figure 8** shows a count of the fungal microflora of the cheese samples a) inoculated with *Penicillium commune* and b) naturally contaminated and treated with the bioactive product of the invention. The statistical test used is ANOVA Tukey with a significance value of p < 0.01.

### EXEMPLARY EMBODIMENTS

### Obtaining the L. plantarum strains

Microorganisms were isolated from cherry tomatoes coming from the University of Valencia toxicology laboratory. For identification purposes, yeast and bacteria were cultured on potato dextrose agar (PDA) and De Man, Rogosa and Sharpe agar (MRS), respectively. Microbial identification was performed by Polymerase Chain Reaction (PCR) amplification and subsequent sequencing of the reference genes for taxonomic purposes. First, genomic DNA was extracted from 24-hour cultures, using the Yeast or Bacteria DNA Preparation Kit (Jena Bioscience) for yeast and bacteria, respectively, following the manufacturer's instructions. The primers used are designed for the 16S rDNA gene, (Ramos-Izquierdo, et al, Isolation, identification and characterisation of lactic acid bacteria for the manufacture of tropical cream cheese). The amplified products were examined by 1% (w/v) agarose gel electrophoresis with Safeview Nucleic Stain (NBS Biologicals) and purified with mi_PCR Purification Kit (Metabion) following the manufacturer's instructions. Sequencing was performed using ABI PRISM BigDye Terminator Cycle Sequencing Ready reagents and the ABI PRISM 3730 sequencer (Applied Biosystems) at the University of Valencia's Central Support Service for Experimental Research (SCSIE). The sequences were manually corrected for sequencing artifacts before comparing them to publicly available sequences in GenBank (www.ncbi.nlm.nih.gov) and the CBS-KNAW website (www.cbs.knaw.nl) for yeast and in EzTaxon and BLAST for bacteria.

The 16S RNA analysis of the bacteria isolated from tomato samples coming from the toxicology laboratory showed that they all belong to the genus *Lactobacillus* and species *plantarum.* All the isolated bacteria used in the study exhibited different genetic characteristics even though they belonged to the same species, and they have been classified as different strains of *Lb. plantarum.* Microorganisms belonging to this genus and species are widely used in agri-food biotechnology due to their extraordinary versatility. Of all the isolated strains, functional experiments were carried out with the deposited strain CECT30089 or BN17.

### Characterisation of the probiotic properties of the bacteria of the strain CECT30089

Characterisation assays of the probiotic properties of the bacteria *Lactobacillus plantarum* BN17 were carried out. The studies focused on the determination of bacterial viability under different conditions of acidic pH and high concentrations of bile salts, capacity for adhesion to CACO-2/TC7 cells, reduced adhesion of *Salmonella* and antibacterial activity against pathogens of the gastrointestinal tract. These experiments were performed independently.

Table 1 shows percentages of viability of *Lactobacillus plantarum* BN17 at acidic pH (2.5-4.5) in a range of 89-158%. At pH 3, 3.5, 4 and 4.5, there is an increase in bacteria compared to the initial inoculum.

The results of tolerance to bile salts (Bile extract porcine), Reference B8631 (Sigma-Aldrich) show that bacteria of the strain CECT30089 can resist the presence of these compounds; moreover, growth continues at concentrations of up to 2% bile salts in the culture medium. From concentrations of 2% bile salts, there is viability close to 90% compared to the initial inoculum, Table 1.

Both bacterial viability at different pHs and resistance to bile salts were determined following a standard protocol in the area.

Regarding assays with CACO-2/TC7 cells, the bacteria of the invention showed a % adhesion of 6.5% and a capacity to reduce the adhesion of *Salmonella* to CACO-2/TC/ cells of 80% (% Adhesion= [Adhered bacteria/Total bacteria inoculated] x 100, Table 1.

The protocol of the experiment was adapted from the one described in patent EP2734057B2, briefly: CACO-2/TC7 human adenocarcinoma cells were cultured in DMEM (Dulbecco's Modified Eagle Medium) supplemented with 10% FCS (*Foetal Calf Serum*), 2 mM L-glutamine and 100 U/ml penicillin/streptomycin in 12-well tissue culture plates at a starting density of 3 × 10⁵ cells/well. After 3 days of culture, the medium was replaced with antibiotic-free DMEM and bacteria of the strain of the invention were added to an MOI (multiplicity of infection) of 30. The plates were prepared in duplicate (each containing 3 replicate wells/treatment) and after 4 hours of incubation at 37°C with 5% CO₂, a pool of cells was lysed with PBS (phosphate buffered saline) containing 1% Triton X-100 and the number of live bacteria was counted using the Miles and Misra method to obtain the total number of bacteria. To obtain the number of bacteria that had invaded the epithelial cells, the second pool of cells was treated with PBS containing 100 pg/ml gentamicin for 2 hours at room temperature before the bacteria were lysed and counted.

Furthermore, *Lactobacillus plantarum* BN17 shows activity against pathogenic fungi and bacteria of the gastrointestinal tract such as *Candida* spp., *Shigella dysenteriae, Listeria monocytogenes, Salmonella enterica, Escherichia coli, Clostridium perfringens, Staphylococcus aureus,* and *Yersinia enterocolitica.* Table 1. The antimicrobial capacity was tested using the inhibition halo test, following a standard protocol for a person skilled in the art.

**Table 1. Characterisation of the probiotic properties of Lactobacillus plantarum strain BN17**

| **Viability at acidic pH (%)** | **pH 2.5** | **pH 3** | **pH 3.5** | **pH 4** | **pH 4.5** |
|---|---|---|---|---|---|
| | 88.6 | 100.7 | 108.7 | 150.3 | 157.7 |
| **Tolerance to bile salts (%)** | **0.5% Bile** | **1% Bile** | **2% Bile** | | |
| | 116.1 | 102.7 | 86.6 | | |
| **Adhesion to CACO-2/TC-7 cells (%)** | 6.5 | | | | |
| **Reduced adhesion of *Salmonella* (%)** | 80 | | | | |
| **Antimicrobial activity against pathogens** | *Candida parapsilosis* | | +++ | | |
| | *Candida glabrata* | | ++ | | |
| | *Candida albicans* | | +++ | | |
| | *Candida guilliermondii* | | ++ | | |
| | *Candida krusei* | | ++ | | |
| | *Candida metapsilosis* | | ++ | | |
| | *Candida auris* C | | ++ | | |
| | *Candida auris* K | | +++ | | |
| | *Candida auris* I | | ++ | | |
| | *Candida auris* B | | +++ | | |
| | *Shigella dysenteriae* | | +++ | | |
| | *Listeria monocytogenes* | | +++ | | |
| | *Salmonella enterica* | | +++ | | |
| | *Escherichia coli* | | +++ | | |
| | *Clostridium perfringens* | | +++ | | |
| | *Staphylococcus aureus* | | +++ | | |
| | *Yersinia enterocolitica* | | +++ | | |

The European Food Safety Authority (EFSA) requires that the microorganisms used to produce certain foods, or which are found therein, as well as for probiotic use, must not have resistance to certain antimicrobial agents of clinical importance. Table 2 shows the results of the minimum inhibitory concentration (MIC) of micrograms of antibiotics per ml of Luria-bertani medium, following the protocol described by EFSA: EFSA Journal 2012;10(6):2740*.* The *Lactobacillus plantarum* BN17 bacteria does not exceed the MIC values recommended by EFSA for each of the antibiotics.

**Table 2. Determination of antibiotic resistance of the Lactobacillus plantarum BN17 bacteria.**

| **Antibiotic** | **Minimum inhibitory concentration (µg/ml)** |
|---|---|
| **Ampicillin** | 1 |
| **Clindamycin** | 2 |
| **Erythromycin** | <0.25 |
| **Gentamicin** | <0.25 |
| **Streptomycin** | 4 |
| **Chloramphenicol** | 8 |
| **Vancomycin** | 32 |
| **Tetracycline** | 32 |
| **Kanamicin** | 8 |

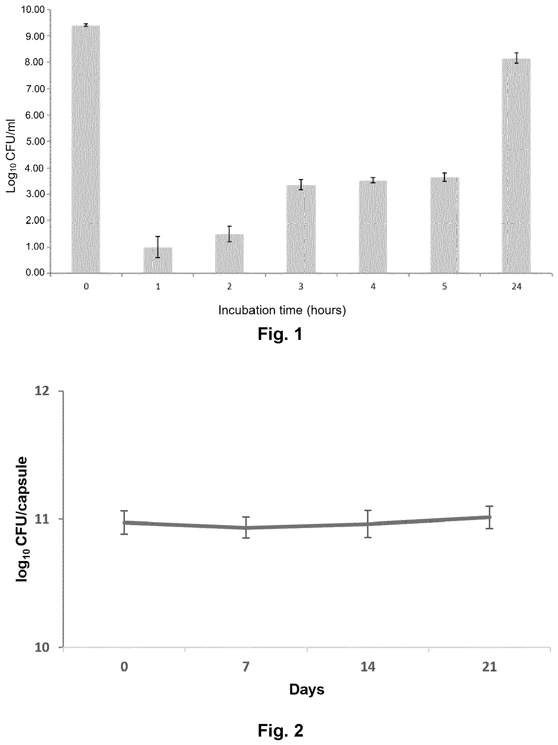

The next step was to simulate the physiological path through the digestive tract. The bacteria of the strain CECT30089 show viability to simulated gastrointestinal digestion (Figure 1), obtaining counts at the end of the digestion of 8 log₁₀ CFU/ml.

### Protocol for the study of resistance to the gastrointestinal tract of Lactobacillus plantarum BN17 using an in vitro dynamic digestion model

*In vitro* dynamic digestion was carried out in the Minifors bioreactor (Bottmingen, Switzerland). Before starting digestion, the pH meter was calibrated and we filled the bioreactor vessel with 940 ml of MRS B medium (Man Rogsa and Sharpe) and 60 ml of artificial saliva, and it was autoclaved in a Selecta autoclave apparatus (Barcelona, Spain) at 121°C for 21 minutes. Subsequently, it was left to cool to 37°C, and we adjusted the pH to 7 and the rotation of the blades to 50 rpm.

Subsequently, we added 10 ml of a 24-h culture at 37°C of Lactobacillus plantarum BN17 to the bioreactor. To simulate stomach conditions, we adjusted the pH of the bioreactor to 2 and added the pepsin solution (5 ml). After incubating for 2 hours, we increased the pH to 6.5 and added the solutions of pancreatin and bile salts (12.5 ml), incubating for 24 h.

Throughout the process, samples were taken on several occasions: immediately after adding the inoculum (0 h), after 1 and 2 h (stomach conditions), and after 3, 4, 5 and 24 h after adding the inoculum (intestinal conditions). 2 to 3 ml were taken with a syringe, serial dilutions were made with peptone water and they were subsequently seeded in petri dishes with MRS Agar for the viable count.

The reagents used were prepared as follows: for pepsin, 1 g of pepsin was diluted in 25 ml of 0.1 N HCl, the solution of pancreatin and bile salts was prepared by diluting 0.1 g of pancreatin and 0.625 g of bile salts in 25 ml of 0.1 N NaHCOs. Artificial saliva was prepared from a 10 ml solution of 89.6 g/l KCl, 10 ml of 20 g/l KSCN, 10 ml of 88.8 g/l NaH₂PO₄, 10 ml of 57 g/l NaSO₄, 1.7 ml of 175.3 g/l NaCl, 20 ml of 84.7 g/l NaHCOs, 8 ml of 25 g/l urea, 290 mg of amylase and 25 mg of mucin. Once mixed, we adjusted the pH to 6.8 and added distilled water until reaching 500 ml.

### Preparation protocol for a gastro-resistant capsule with Lactobacillus plantarum BN17

To make the lyophilisate to be introduced into the capsule, we started from a culture of *Lactobacillus plantarum* BN17 at 37°C for 24 h in MRS broth. After incubation was complete, the contents were poured into 15 ml tubes and centrifuged at 4000 rpm for 10 minutes, the pellet was washed with 2 ml PBS and centrifuged again. Lastly, the bacteria were suspended in 20% ultrapasteurised skimmed milk (UHT), and at a final suspension of bacteria comprised between 10⁴ and 10¹⁵ CFU/ml, they were frozen at -80°C and lyophilised for 72 h in the FreeZone 2.5 L lyophiliser (Labconco, Kansas City, MO, USA). Once the lyophilisate of *Lactobacillus plantarum* BN17 was obtained, the capsules were prepared by using a 100-capsule manual encapsulator following a standard state-of-the-art protocol.

After the viability studies of the lyophilised probiotic stored in a gastro-resistant capsule (Figure 2), we can observe a constant viable count during 21 days of storage at 20°C. Therefore, the stability of *Lactobacillus plantarum* BN17 lyophilised and encapsulated for a long period of time is ensured.

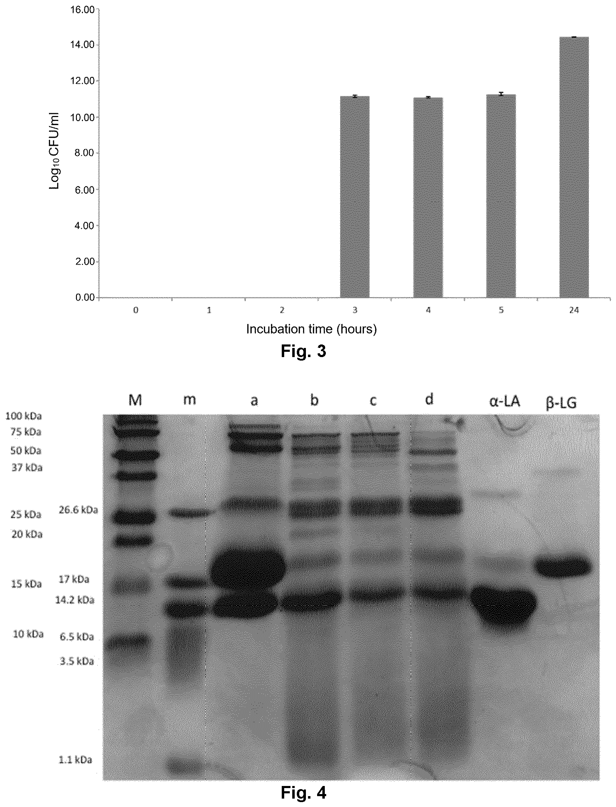

Furthermore, the previously described protocol of resistance to the gastrointestinal tract was repeated, but this time by using the capsule with lyophilised Lactobacillus plantarum BN17. This experiment showed a release of the bacteria from the inside of the capsule after 3 h of digestion, reaching counts of 14 log₁₀ CFU/ml at the end of the same (Figure 3). Higher yields than when administering the bacteria without encapsulation (Figure 1). In the interval of 0-3 hrs, no growth was observed due to the slow release of the capsule contents.

### Method for obtaining an antimicrobial bioactive product

### Whey fermentation by the strain CECT30089

Sweet goat whey provided by the company ALCLIPOR, S.A.L. (Benassal, Spain) is fermented with CECT30089 bacteria (at 37°C) for 72 hours. For each condition, 2 litres of sweet whey were used, with a 20 ml inoculum at a concentration of 10⁹CFU/ml of the inoculum volume.

To eliminate possible microorganisms that may affect the fermentation process, goat whey is pasteurised at 65°C for 30 minutes and then fermented under sterile conditions with the previously activated starter culture. Activation is carried out by incubating a starter culture or inoculum in the whey so that the bacteria are activated; this substrate must be identical to that of the subsequent fermentation. Once activated, a 1:100 scale is made, with a concentration of 10⁹CFU/ml of the inoculum volume.

After fermentation, the samples are centrifuged for 10 minutes at 4000 rpm to eliminate microorganisms and 1 mM phenylmethanesulfonyl fluoride protease inhibitor is added to the supernatant. The fermented whey samples are lyophilised and stored at -80°C for subsequent analysis.

### Isolation and characterisation of antimicrobial bioactive compounds

Phenolic compounds are extracted using the method described by Brosnan et al. (2014) (The QuEChERS approach in a novel application for the identification of antifungal compounds produced by lactic acid bacteria cultures. Talanta, 129, 364-373.) with some modifications. Ten millilitres of fermented whey were extracted using the QuEChERS method. Lastly, the sample is suspended in 1 ml of water- acetonitrile (90:10 v/v), filtered through a 0.22 µm nylon filter and stored for chromatographic analysis. For chromatographic determination, an Agilent 1200-LC system equipped with a vacuum degasser, autosampler and binary pump was used. Separation by chromatography is performed using a Gemini C18 column. MS analyses are performed using an Agilent 6540 High Definition Precision q-TOF-MS coupled to HPLC, equipped with a Dual Agilent Jet Stream Electrospray Ionisation Interface (Dual AJS ESI) in negative ionisation mode. Data interpretations are performed using MassHunter workstation software (Agilent Technologies).

The analytical data collected showed that the bacteria of the strain CECT30089 were responsible for producing antimicrobial molecules known globally as biocomplexes, the observed antifungal effect is not associated with a specific molecule. In the biocomplex, phenylactic acid (PLA) has been identified, a phenolic acid with high antimicrobial properties, in concentrations greater than 200 mg/l, and another 19 different compounds therebetween, ferulic acid, caffeic acid, syringic acid, benzoic acid, gallic acid, etc., all of them being elements responsible for the antifungal and antibacterial effect. The concentrations of the compounds mentioned in the antimicrobial formulation varied, between 10 and 250 mg/l.

### Isolation and characterisation of antimicrobial bioactive peptides

The degree of protein hydrolysis during fermentation performed by the isolated microorganisms is analysed by SDS-PAGE using a 15% (w/v) separating gel and a 4% stacking gel. To display the protein bands, the gels were stained in a staining solution: 0.1% Brilliant Blue R-250, 50% water, 40% methanol and 10% acetic acid. The molecular mass of proteins is evaluated by comparing it with BioRad Precision Plus Protein standards (high MW range) and natural polypeptide SDS-PAGE (low MW range) (Luz et al., 2018, Evaluation of biological and antimicrobial properties of freeze-dried whey fermented by different strains of Lactobacillus plantarum, Food Funct, 9, 3688-3697).

Peptide purification from fermented whey is carried out using the method described by Saladino et al. (2016) In vitro antifungal activity of lactic acid bacteria against mycotoxigenic fungi and their application in loaf bread shelf life improvement with some modifications. The fraction containing peptides below 3 kDa is analysed by LC-ESI-MS-TOF. The LC system used for chromatographic determination was an Agilent 1200 system, equipped with a vacuum degasser, autoinjector and binary pump. The column is a bioZen C18 measuring 50 x 2.1 mm and having a particle size of 2.6 µm. Mass spectrometry analysis is carried out using an Agilent 6540 Ultra High Definition Accurate Mass Quadrupole Time-of-Flight (Q-TOF) system, equipped with a Dual Agilent Jet Stream Electrospray Ionisation Interface (Dual AJS ESI) in MS positive ionisation mode. The generated MS spectrum is processed for de novo peptide sequencing using MassHunter B.08.00 qualitative analysis software (Agilent).

**Table 3. Antimicrobial bioactive peptides identified in whey fermented by LAB of the strain CECT30089.**

| **no. Protein** | **Observed MS (Da)** | **Expected MS (Da)** | **Delta** | **Peptide** |
|---|---|---|---|---|
| 1 α-lactalbumin | 618.7119 | 618.3457 | 0.36 | EQLTK |
| 2 α-lactalbumin | 1065.1777 | 1064.5047 | 0.67 | GYGGVSLPEW |
| 3 α-lactalbumin | 570.6892 | 570.2592 | 0.43 | VCTTF |
| 4 α-lactalbumin | 650.7763 | 650.3178 | 0.45 | ALCSEK |
| 5 α-lactalbumin | 957.0154 | 956.3891 | 0.61 | CKDDQNPH |
| 6 β-lactoglobulin | 696.7854 | 696.3352 | 0.45 | VAGTWY |
| 7 β-lactoglobulin | 976.0777 | 975.4993 | 0.57 | AASDISLLDA |
| 8 β-lactoglobulin | 674.8664 | 674.4236 | 0.44 | IPAVFK |
| 9 K-casein | 1202.4103 | 1201.6800 | 0.73 | YQRRPAIAIN |
| 10 Lactoferrin (LF) | 1304.5316 | 1303.6913 | 0.84 | GRRRRSVQWC |
| 11 Lactoferrin (LF) | 1397.6768 | 1396.6725 | 1.00 | KCFQWQRNMR |
| 12 Lactoferrin (LF) | 1056.3234 | 1055.6030 | 0.72 | KVRGPPVSCI |
| 13 Lactoferrin (LF) | 1302.5999 | 1301.7372 | 0.86 | WQRRMRKLGA |
| 14 Lactoferrin (LF) | 1231.4740 | 1230.6524 | 0.82 | APRKNVRWCT |
| 15 Lactoferrin (LF) | 1355.5238 | 1354.5885 | 0.93 | PEWSKCYQWQ |
| 16 Lactoferrin (LF) | 1172.4498 | 1171.6841 | 0.76 | RRMRKLGAPS |
| 17 Lactoferrin (LF) | 1007.2070 | 1006.5462 | 0.66 | ITCVRRTSA |
| 18 Lactoferrin (LF) | 1497.8442 | 1496.7514 | 1.09 | FKCRRWQWRM |
| 19 Lactoferrin (LF) | 1018.2711 | 1017.5761 | 0.69 | KKLGAPSITC |
| 20 Lactoferrin (LF) | 1046.2846 | 1045.5823 | 0.70 | RKLGAPSITC |
| 21 Lactoferrin (LF) | 618.7178 | 618.3682 | 0.34 | VRRTS |
| 22 Lactoferrin (LF) | 1110.2818 | 1109.5296 | 1.05 | VSQPEATKCF |
| 23 Lactoferrin (LF) | 1072.2791 | 1071.5615 | 0.71 | GPPVSCIKRD |
| 24 Lactoferrin (LF) | 1238.4160 | 1237.5670 | 0.84 | PEATKCFQWQ |
| 25 Lactoferrin (LF) | 960.1995 | 959.5680 | 0.63 | RNMRKVR |
| 26 Lactoferrin (LF) | 1386.6504 | 1385.6565 | 0.99 | SKCYQWQRRM |
| 27 Lactoferrin (LF) | 1046.2846 | 1045.5823 | 0.70 | RKLGAPSITC |

Protein analysis performed by SDS-PAGE electrophoresis of whey fermented by lactic acid bacteria (Figure 4) has shown that the bacteria of the strain used (BN17) have better proteolytic activity compared to the samples treated with other strains (BN16 and E4) as observed in the reduced intensity of the bands that characterise the control (a). As shown in the electrophoretic analysis, the two proteins that show a high degree of hydrolysis are α-lactalbumin and β-lactoglobulin, the two main soluble proteins that characterise whey from the point of view of protein.

The purified peptides (27 different antimicrobial peptides, Table 3) exhibit a variable amino acid composition between 5 and 10 compounds with a variable molecular weight between 570 and 1496 Da. The identified peptides were purified on a large scale by using the purification methodology through preparative C18 column chromatography, coupled to the detection in series of diodes for the identification of the analysed compounds. The detector output is connected to a preparative fraction collector for purifying volumes greater than 50 ml.

### Antimicrobial activity of whey fermented by bacteria of the strain CECT30089

To evaluate the activity of the antimicrobial formulation fermented by the lactic bacteria of the strain CECT30089, the mycotoxigenic strains of *P*. *camemberti* CECT 2267, *P. roqueforti* CECT 2905, *P. expansum* CECT 2278, *P*. *digitatum* CECT 2954, *P. brevicompactum* CECT 2316, and *P. commune* CECT 20767, provided by the Spanish Type Culture Collection (CECT), were used. For the qualitative assessment of antimicrobial activity, the method by Torrijos et al., (2019) (Development of a Bioactive Sauce Based on Oriental Mustard Flour with Antifungal Properties for Pita Bread Shelf Life Improvement. Molecules, 24, 1019) was used. The determination of the quantitative antimicrobial activity was carried out according to the method described by Fothergill et al (2012) (Antifungal Susceptibility 521 Testing: Clinical Laboratory and Standards Institute (CLSI) Methods. In G. Hall (Ed.), Interactions of Yeasts, Moulds, and Antifungal Agents. Totowa: Humana Press.) with some modifications described below. A volume of fermented whey with final concentrations ranging from 0.5 to 250 mg/ml was added in sterile 96-well microplates. After that, the microwells were contaminated with a suspension of toxigenic fungi. The positive control consisted of medium contaminated with non-hydrolysed goat whey and the negative control was non-contaminated medium without any treatment. After the incubation time, the concentration of antimicrobial formulation where no visible fungal growth was observed was considered the minimum inhibitory concentration (MIC). After that, the concentration of MIC and concentration above MIC were cultured on PDA plates for the determination of the minimum fungicidal concentration (MFC). After incubation, the MFC value was the concentration where there was no visible fungal growth.

**Table 4. Antimicrobial activity of the formulation by the L. plantarum BN17 strain, CECT30089.**

| Fungus | Strain | BN17 | |
|---|---|---|---|
| | | MIC (mg/ml) | MIF (mg/ml) |
| ***Penicillium camemberti*** | **CECT 2267** | 12.5 | >100 |
| ***Penicillium expansum*** | **CECT 2278** | 12.5 | >100 |
| ***Penicillium brevicopactum*** | **CECT 2316** | 12.5 | >100 |
| ***Penicillium commune*** | **CECT 20767** | 50 | >100 |

| Fungus | Strain | BN17 |
|---|---|---|
| ***Penicillium camemberti*** | **CECT 2267** | ++ |
| ***Penicillium expansum*** | **CECT 2278** | + |
| ***Penicillium brevicopactum*** | **CECT 2316** | + |
| ***Penicillium commune*** | **CECT 20767** | + |

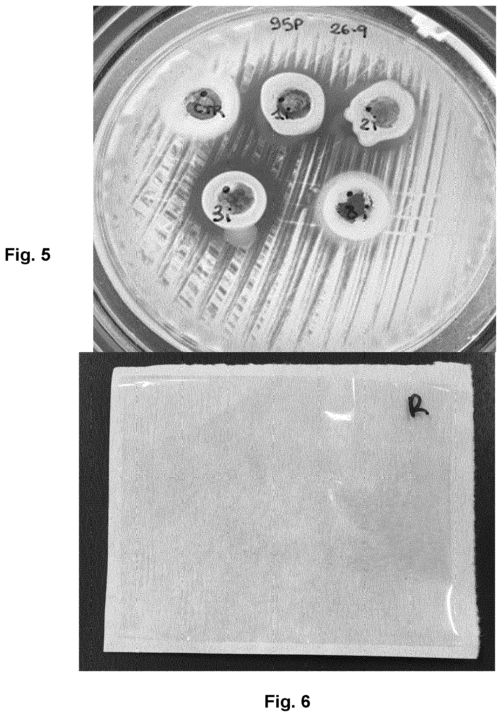

As shown by the analysis of the results highlighted in Table 4, the antimicrobial formulation of the deposited *L. plantarum* strain was active against all assayed fungi, see Figure 5 and Table 4.

The results of the microbial activity in liquid medium of the antimicrobial formulations fermented by the lactic acid bacteria of the deposited strain showed minimum inhibitory doses (MIC) and minimum fungicidal doses (MFC) varying between 12.5 and 100 mg/ml, confirming the results obtained and observed in solid PDA medium. These last results are extremely interesting if we consider that the inhibitory doses are in the range of milligrams per millilitre, very low concentrations since it is a biocomplex of natural substances produced through microbial fermentation. This exhibits very low MIC values compared to the most characteristic fungi that contaminate cheese and that also develop in many foods in the preservation phase, such as *P. camemberti, P. expansum,* which is quite ubiquitous in nature due to its ability to contaminate different types of both animal and plant products, and *P. brevicompactum.*

### Comparison with other L. plantarum strains

The strain BN17 exhibited greater antifungal activity than other *L*. *plantarum* strains previously deposited in the Spanish Type Culture Collection:

**Table 5. Comparative assay of MIC and MIF of the strain object of the invention (BN17) with respect to other L. plantarum strains**

| **Fungus** | **Strain** | **BN17 (CECT30089)** | | **5L1 (CECT 223)** | | **5H1 (CECT 220)** | | **1F1 (CECT 221)** | | **1L1 (CECT 748)** | |
|---|---|---|---|---|---|---|---|---|---|---|---|
| | | **MIC (mg/ml)** | **MIF (mg/ml)** | **MIC (mg/ml)** | **MIF (mg/ml)** | **MIC (mg/ml)** | **MIF (mg/ml)** | **MIC (mg/ml)** | **MIF (mg/ml)** | **MIC (mg/ml)** | **MIF (mg/ml)** |
| ***Penicillium camemberti*** | **CECT 2267** | 12.5 | >100 | 25 | >100 | 50 | >100 | 50 | >100 | >100 | >100 |
| ***Penicillium expansum*** | **CECT 2278** | 12.5 | >100 | 25 | >100 | 50 | >100 | 50 | >100 | 100 | >100 |
| ***Penicillium brevicopactum*** | **CECT 2316** | 12.5 | >100 | 25 | >100 | 50 | >100 | 50 | >100 | 100 | >100 |
| ***Penicillium commune*** | **CECT 20767** | 50 | >100 | 100 | >100 | >100 | >100 | >100 | >100 | >100 | >100 |
| ***Penicillium roqueforti*** | **CECT 2905** | 25 | >100 | 50 | >100 | 50 | >100 | 50 | >100 | >100 | >100 |
| ***Penicillium verrucosum*** | **VTT D-01847** | 25 | >100 | 50 | >100 | 100 | >100 | 100 | >100 | 100 | >100 |
| ***Penicillium digitatum*** | **CECT 2954** | 50 | >100 | 50 | >100 | 100 | >100 | 100 | >100 | >100 | >100 |
| ***Penicillium solitum*** | **CECT 20818** | 12.5 | >100 | 25 | >100 | 100 | >100 | 50 | >100 | >100 | >100 |
| ***Penicillium nordicum*** | **CECT 2320** | 25 | >100 | 50 | >100 | >100 | >100 | 50 | >100 | >100 | >100 |
| ***Penicillium pinophilum*** | **CECT 2912** | 12.5 | >100 | 50 | >100 | 100 | >100 | 50 | >100 | >100 | >100 |
| ***Aspergillus parasiticus*** | **CECT 2681** | 50 | >100 | 100 | >100 | >100 | >100 | >100 | >100 | >100 | >100 |
| ***Aspergillus steynii*** | **CECT 20510** | 50 | >100 | 100 | >100 | 100 | >100 | >100 | >100 | >100 | >100 |
| ***Aspergillus flavus*** | **ISPA 8111** | 50 | >100 | 100 | >100 | 100 | >100 | >100 | >100 | 100 | >100 |
| ***Aspergillus carbonarius*** | **ISPA 5010** | 25 | >100 | 100 | >100 | 100 | >100 | 100 | >100 | >100 | >100 |
| ***Aspergillus niger*** | **CECT 2088** | 50 | >100 | >100 | >100 | >100 | >100 | >100 | >100 | >100 | >100 |
| ***Aspergillus brasiliensis*** | **CECT 2574** | 50 | >100 | >100 | >100 | >100 | >100 | >100 | >100 | >100 | >100 |
| ***Aspergillus sclerotioniger*** | **CECT 20583** | 25 | >100 | 100 | >100 | 100 | >100 | 100 | >100 | >100 | >100 |
| ***Aspergillus tubingensis*** | **CECT 20543** | 50 | >100 | 100 | >100 | 100 | >100 | >100 | >100 | >100 | >100 |
| ***Aspergillus tubingensis*** | **CECT 20544** | 50 | >100 | 100 | >100 | >100 | >100 | 100 | >100 | >100 | >100 |
| ***Aspergillus lacticoffeatus*** | **CECT 20581** | 50 | >100 | 100 | >100 | 100 | >100 | 100 | >100 | >100 | >100 |
| ***Fusarium moniliforme*** | **CECT 2152** | 12.5 | 50 | 50 | 100 | 50 | 100 | 100 | >100 | 100 | >100 |
| ***Fusarium moniliforme*** | **CECT 2987** | 25 | 50 | 50 | 100 | 100 | >100 | 100 | >100 | >100 | >100 |
| ***Fusarium graminearum*** | **ISPA 6352** | 25 | 50 | 50 | 100 | 50 | 100 | 50 | 100 | 100 | >100 |
| ***Fusarium proliferatum*** | **ISPA 12101** | 12.5 | 50 | 50 | 100 | 50 | 100 | 100 | >100 | 100 | >100 |
| ***Fusarium verticillioides*** | **ISPA 5850** | 12.5 | 50 | 50 | >100 | 100 | >100 | 100 | >100 | >100 | >100 |
| ***Fusarium verticillioides*** | **ISPA 5845** | 25 | 50 | 50 | 10 | 50 | 10 | 100 | >100 | >100 | >100 |
| ***Fusarium verticillioides*** | **ISPA 12044** | 25 | 50 | 50 | 100 | 50 | 100 | 50 | 100 | 100 | >100 |
| ***Fusarium sporotrichioides*** | **ITEM 12168** | 25 | 50 | 50 | >100 | 100 | >100 | 100 | >100 | >100 | >100 |
| ***Fusarium langsethiae*** | **ITEM 11031** | 12.5 | 50 | 25 | 100 | 50 | 100 | 100 | >100 | 100 | >100 |
| ***Fusarium poae*** | **ITEM 9131** | 12.5 | 50 | 25 | 100 | 25 | 100 | 100 | >100 | 100 | >100 |

The best probiotic activity of the strain BN17 is due to the fact that it exhibits better properties than other *L. plantarum* strains when determining resistance to acidic pH, tolerance to bile salts, % adhesion and % reduced adhesion of *Salmonella* to CACO2-TC-7 cells, as well as antimicrobial activity against pathogens in a solid medium: BN17 (**Table 6**), 5L1 (**Table 7**), 5H1 (**Table 8**), 1F1 (**Table 9**) and 1L1 (**Table 10**)

**Table 6. Characterisation of the probiotic properties of the Lactobacillus plantarum BN17 strain**

| **Viability at acidic pH (%)** | **pH 2.5** | **pH 3** | **pH 3.5** | **pH 4** | **pH 4.5** |
|---|---|---|---|---|---|
| | 88.6 | 100.7 | 108.7 | 150.3 | 157.7 |
| **Tolerance to bile salts (%)** | **0.5% Bile** | **1% Bile** | **2% Bile** | | |
| | 116.1 | 102.7 | 86.6 | | |
| **Adhesion to CACO-2/TC-7 cells (%)** | 6.5 | | | | |
| **Reduced adhesion of *Salmonella* (%)** | 80 | | | | |
| **Antimicrobial activity against pathogens (Inhibition halo in mm)** | *Candida parapsilosis* | | 12 | | |
| | *Candida glabrata* | | 10 | | |
| | *Candida albicans* | | 12 | | |
| | *Candida guilliermondii* | | 10 | | |
| | *Candida krusei* | | 10 | | |
| | *Candida metapsilosis* | | 10 | | |
| | *Candida auris* C | | 10 | | |
| | *Candida auris* K | | 12 | | |
| | *Candida auris* I | | 10 | | |
| | *Candida auris* B | | 12 | | |
| | *Shigella dysenteriae* | | 12 | | |
| | *Listeria monocytogenes* | | 12 | | |
| | *Salmonella enterica* | | 12 | | |
| | *Escherichia coli* | | 12 | | |
| | *Clostridium perfringens* | | 12 | | |
| | *Staphylococcus aureus* | | 12 | | |
| | *Yersinia enterocolitica* | | 12 | | |

**Table 7. Characterisation of the probiotic properties of the Lactobacillus plantarum 5L1 strain**

| **Viability at acidic pH (%)** | **pH 2.5** | **pH 3** | **pH 3.5** | **pH 4** | **pH 4.5** |
|---|---|---|---|---|---|
| | 60.2 | 73.6 | 84.7 | 91.4 | 105.7 |
| **Tolerance to bile salts (%)** | **0.5% Bile** | **1% Bile** | **2% Bile** | | |
| | 91.7 | 71.5 | 52.8 | | |
| **Adhesion to CACO-2/TC-7 cells (%)** | 2.1 | | | | |
| **Reduced adhesion of *Salmonella* (%)** | 38 | | | | |
| **Antimicrobial activity against pathogens (Inhibition halo in mm)** | *Candida parapsilosis* | | 7 | | |
| | *Candida glabrata* | | 8 | | |
| | *Candida albicans* | | 9 | | |
| | *Candida guilliermondii* | | 5 | | |
| | *Candida krusei* | | 5 | | |
| | *Candida metapsilosis* | | 6 | | |
| | *Candida auris* C | | 5 | | |
| | *Candida auris* K | | 5 | | |
| | *Candida auris* I | | 6 | | |
| | *Candida auris* B | | 5 | | |
| | *Shigella dysenteriae* | | 7 | | |
| | *Listeria monocytogenes* | | 7 | | |
| | *Salmonella enterica* | | 7 | | |
| | *Escherichia coli* | | 8 | | |
| | *Clostridium perfringens* | | 7 | | |
| | *Staphylococcus aureus* | | 7 | | |
| | *Yersinia enterocolitica* | | 7 | | |

**Table 8. Characterisation of the probiotic properties of the Lactobacillus plantarum 5H1 strain**

| **Viability at acidic pH (%)** | **pH 2.5** | **pH 3** | **pH 3.5** | **pH 4** | **pH 4.5** |
|---|---|---|---|---|---|
| | 28.5 | 44.8 | 67.9 | 81.1 | 90.5 |
| **Tolerance to bile salts (%)** | **0.5% Bile** | **1% Bile** | **2% Bile** | | |
| | 71.5 | 35.4 | 18.5 | | |
| **Adhesion to CACO-2/TC-7 cells (%)** | 1.7 | | | | |
| **Reduced adhesion of *Salmonella* (%)** | 18 | | | | |
| **Antimicrobial activity against pathogens (Inhibition halo in mm)** | *Candida parapsilosis* | | 6 | | |
| | *Candida glabrata* | | 5 | | |
| | *Candida albicans* | | 6 | | |
| | *Candida guilliermondii* | | 0 | | |
| | *Candida krusei* | | 0 | | |
| | *Candida metapsilosis* | | 6 | | |
| | *Candida auris* C | | 0 | | |
| | *Candida auris* K | | 0 | | |
| | *Candida auris* I | | 5 | | |
| | *Candida auris* B | | 5 | | |
| | *Shigella dysenteriae* | | 6 | | |
| | *Listeria monocytogenes* | | 5 | | |
| | *Salmonella enterica* | | 5 | | |
| | *Escherichia coli* | | 6 | | |
| | *Clostridium perfringens* | | 5 | | |
| | *Staphylococcus aureus* | | 7 | | |
| | *Yersinia enterocolitica* | | 7 | | |

**Table 9. Characterisation of the probiotic properties of the Lactobacillus plantarum 1F1 strain**

| **Viability at acidic pH (%)** | **pH 2.5** | **pH 3** | **pH 3.5** | **pH 4** | **pH 4.5** |
|---|---|---|---|---|---|
| | 42.8 | 68.7 | 84.1 | 94.7 | 98.8 |
| **Tolerance to bile salts (%)** | **0.5% Bile** | **1% Bile** | **2% Bile** | | |
| | 89.5 | 74.5 | 66.8 | | |
| **Adhesion to CACO-2/TC-7 cells (%)** | 2.8 | | | | |
| **Reduced adhesion of *Salmonella* (%)** | 21 | | | | |
| **Antimicrobial activity against pathogens (Inhibition halo in mm)** | *Candida parapsilosis* | | 0 | | |
| | *Candida glabrata* | | 0 | | |
| | *Candida albicans* | | 0 | | |
| | *Candida guilliermondii* | | 0 | | |
| | *Candida krusei* | | 0 | | |
| | *Candida metapsilosis* | | 0 | | |
| | *Candida auris* C | | 0 | | |
| | *Candida auris* K | | 0 | | |
| | *Candida auris* I | | 0 | | |
| | *Candida auris* B | | 0 | | |
| | *Shigella dysenteriae* | | 5 | | |
| | *Listeria monocytogenes* | | 5 | | |
| | *Salmonella enterica* | | 5 | | |
| | *Escherichia coli* | | 6 | | |
| | *Clostridium perfringens* | | 5 | | |
| | *Staphylococcus aureus* | | 5 | | |
| | *Yersinia enterocolitica* | 5 | | | |

**Table 10. Characterisation of the probiotic properties of the Lactobacillus plantarum 1L1 strain**

| **Viability at acidic pH (%)** | **pH 2.5** | **pH 3** | **pH 3.5** | **pH 4** | **pH 4.5** |
|---|---|---|---|---|---|
| | 62.7 | 76.9 | 91.2 | 95.6 | 99.3 |
| **Tolerance to bile salts (%)** | **0.5% Bile** | **1% Bile** | **2% Bile** | | |
| | 91.5 | 85.6 | 67.8 | | |
| **Adhesion to CACO-2/TC-7 cells (%)** | 3.1 | | | | |
| **Reduced adhesion of *Salmonella* (%)** | 35 | | | | |
| **Antimicrobial activity against pathogens (Inhibition halo in mm)** | *Candida parapsilosis* | | 5 | | |
| | *Candida glabrata* | | 6 | | |
| | *Candida albicans* | | 6 | | |
| | *Candida guilliermondii* | | 0 | | |
| | *Candida krusei* | | 5 | | |
| | *Candida metapsilosis* | | 5 | | |
| | *Candida auris* C | | 5 | | |
| | *Candida auris* K | | 6 | | |
| | *Candida auris* I | | 5 | | |
| | *Candida auris* B | | 0 | | |
| | *Shigella dysenteriae* | | 5 | | |
| | *Listeria monocytogenes* | | 6 | | |
| | *Salmonella enterica* | | 6 | | |
| | *Escherichia coli* | | 6 | | |
| | *Clostridium perfringens* | | 0 | | |
| | *Staphylococcus aureus* | | 0 | | |
| | *Yersinia enterocolitica* | | 0 | | |

Finally, the strain BN17 also stood out due to its low resistance to antimicrobial antibiotics, a requirement for any probiotic product (**Table 11**).

**Table 11. Determination of antibiotic resistance of Lactobacillus plantarum strains**

| **Antibiotic** | **Minimum Inhibitory Concentration (µg/ml)** | | | | |
|---|---|---|---|---|---|
| | **BN17** | **5L1** | **5H1** | **1F1** | **1L1** |
| **Ampicillin** | 1 | 4 | 4 | 8 | 4 |
| **Clindamycin** | 2 | 8 | 32 | 32 | 8 |
| **Erythromycin** | <0.25 | 2 | 4 | 8 | 4 |
| **Gentamicin** | <0.25 | 2 | 2 | 2 | 4 |
| **Streptomycin** | 4 | 16 | 16 | 32 | 16 |
| **Chloramphenicol** | 8 | 32 | 32 | 32 | 32 |
| **Vancomycin** | 32 | 64 | 128 | 128 | 64 |
| **Tetracycline** | 32 | 64 | 128 | 128 | 64 |
| **Kanamicin** | 8 | 16 | 16 | 32 | 16 |

Considering the probiotic properties of the strain BN17 compared to other *L. plantarum* strains in tables 5-11, and the evidence of high production of antimicrobial bioactive molecules described in the previous paragraphs, it was decided to continue with this microorganism to develop the demonstrator, and for its manufacturing and validation process.

### Definition of the bioactive product. Materials and manufacturing process.

To obtain a bioactive product, the cheese demonstrator packaging format has been selected as an example. Based on the current packaging that has been found on the market, it is considered that the packaging must be in direct contact with the cheese to maximise the effectiveness of the coating with the antimicrobial formulation, and for this reason, a preferred embodiment of the invention are separators or films for cheese slices, in order to maximise contact between the active compound and the cheese.

In this particular example, a commercial food separator film was used on which the antimicrobial whey coating formulation was applied.

### Antimicrobial whey coating formulation.

Step a) comprises the fermentation of sweet goat whey at 37°C for 72 hours, with an inoculum of 10⁹CFU/ml of the inoculum volume with the previously activated strain CECT30089. After fermentation, the samples are centrifuged for 10 minutes at 4000 rpm to eliminate microorganisms and the antimicrobial ferment is lyophilised; lyophilisation is carried out following a standard and known protocol in the sector. The antimicrobial formulation was dissolved in water at a concentration of between 15-20% by weight. The solubility of the antimicrobial formulation in water is directly related to the nature of the starting substrate; if the protein content is very high, the dissolution of the antimicrobial ferment in water will be more difficult and lower concentrations will be reached.

For step b) of the method, PVOH was selected as the polymeric matrix suitable for direct contact with food, said matrix being dissolved in water, thus obtaining a concentration of between 15% and 20% of polymeric matrix in water.

In step c) the dissolved antimicrobial formulation (a) was added to the dissolved polymeric matrix (b) to prepare the antimicrobial whey coating formulation, at a concentration of 20% by weight of antimicrobial formulation over the total. The final formulation obtained is acidified until reaching a pH of 3 with 4.76% phosphoric acid in water.

### Application of the coating

The coating formulations obtained were applied (step d) by means of wand application equipment. Wand 3 was used, which applies 24 µm of wet solution in K Control Coater 101 equipment, following a standard protocol in the technical sector, on PET, paper or OPP/EVOH/OPP film, to obtain film and active separators.

Figure 6 shows the image of the final bioactive product or bioactive film obtained by the method of the invention.

### Use of the bioactive product

The effectiveness of the product obtained as a result of the foregoing method was validated in sliced mozzarella cheese without preservatives. First, the efficacy of the antimicrobial film was assayed in a Petri dish with the main cheese contaminants and derivatives, and its efficacy was demonstrated against most of the fungi assayed, Table 12. Its great activity against the two main fungi that contaminate cheese, such as *P. camemberti* and especially *P. commune,* should be noted.

**Table 12. Antimicrobial activity in solid media of the antimicrobial film containing the antimicrobial whey coating formulation obtained from L. plantarum CECT30089/BN17.**

| Fungus | Strain | CECT30089/ BN17 |
|---|---|---|
| *Penicillium camemberti* | CECT 2267 | ++ |
| *Penicillium expansum* | CECT 2278 | + |
| *Penicillium roqueforti* | CECT 2905 | + |
| *Penicillium brevicopactum* | CECT 2316 | + |
| *Penicillium commune* | CECT 20767 | ++ |
| *Penicillium verrucosum* | VTT D 01847 | + |

Secondly, the antimicrobial device was applied for its direct validation in cheeses. The antimicrobial whey coating formulation from whey fermented by the BN17 bacterium was used to cover the plastic films/separators through the process described in previous sections, obtaining a bioactive product for its direct application on sliced cheese. The bioactive film was applied to a cheese inoculated with 10⁵ spores/g of the previously demonstrated fungal species. As a control, mozzarella cheese contaminated with the same fungal species and treated with a whey-free plastic film was used. The samples were kept at a refrigerated temperature of 4°C for 30 days.

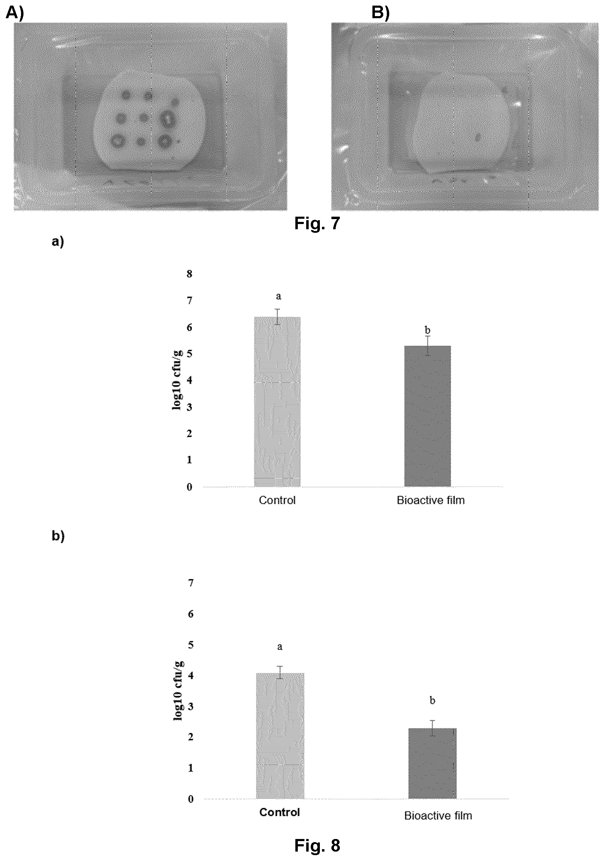

As shown in Figure 7, having reached the established incubation time, the control sample shows a very widespread growth of the fungal population, while the sample treated with the antimicrobial film (bioactive film) did not show any sign of fungal contamination.

Table 13 shows the results of the shelf life of the cheese inoculated with 10⁵ spores/g of *Penicillium commune* and naturally contaminated and treated with bioactive film compared to the control cheese, treated with a commercial plastic film. Inoculation with *P. commune* was performed following a standard protocol in the technical sector.

The use of the antimicrobial film showed an increase in the shelf life of the cheese inoculated with *Penicillium commune* and naturally contaminated compared to the control at 2 and 4 days, respectively. In other words, an increase of 14% and 21% of the shelf life of the cheese.

Subsequently, the microbiological count of the cheese was evaluated in both conditions, and for this, homogenisation and serial dilutions with 0.1% peptone water with 0.1% Tween 80 were carried out. Finally, 100 µl of these dilutions were seeded on plates with PCA (Plate Count Agar) culture medium and incubated at 26°C for 48-72 h to count viable microorganisms. (Quiles, J. M., Manyes, L., Luciano, F., Mañes, J., & Meca, G. (2015). Influence of the antimicrobial compound allyl isothiocyanate against the Aspergillus parasiticus growth and its aflatoxins production in pizza crust. Food and Chemical Toxicology, 83, 222-228.). The bioactive film reduced the growth of *Penicillium commune* and the normal microflora of the cheese compared to the controls of 1.1 and 1.8 log CFU/g, respectively (Figure 8). This indicates that the product of the invention can inhibit or delay the fungal growth of numerous species and not only of *P. commune.*

**Table 13. Shelf life study of cheese a) inoculated with Penicillium commune and b) naturally contaminated.**

| **a)** | | | | | | | | | | | | | | | | | | |
|---|---|---|---|---|---|---|---|---|---|---|---|---|---|---|---|---|---|---|
| **Samples** | **Growth retention time (days)** | | | | | | | | | | | | | | | | | |
| | 1 | 2 | 3 | 4 | 5 | 6 | 7 | 8 | 9 | 10 | 11 | 12 | 13 | 14 | 15 | 16 | 17 | 18 |
| **Control** | - | - | - | - | - | - | - | - | - | - | - | - | - | - | + | + | + | + |
| **Bioactive film** | - | - | - | - | - | - | - | - | - | - | - | - | - | - | - | - | + | + |

| **b)** | **Growth retention time (days)** | | | | | | | | | | | | | | | | | |
|---|---|---|---|---|---|---|---|---|---|---|---|---|---|---|---|---|---|---|
| **Samples** | | | | | | | | | | | | | | | | | | |
| | 1 | 2 | 3 | 4 | 5 | 6 | | 7 | 3 | 9 | 10 | 11 | 12 | 13 | 14 | 15 | 16 | 17 |
| **Control** | - | - | - | - | - | | - | - | - | - | - | - | - | - | - | - | - | - |
| **Bioactive film** | - | - | - | - | - | | - | - | - | - | - | - | - | - | - | - | - | - |

| **Samples** | **Growth retention time (days)** | | | | | | | | | | | | | | | | | |
|---|---|---|---|---|---|---|---|---|---|---|---|---|---|---|---|---|---|---|
| | | | 18 | | 19 | | | 20 | | 21 | | 22 | 23 | 24 | | 25 | 26 | |
| **Control** | | | - | | - | | | + | | + | | + | + | + | | + | + | |
| **Bioactive film** | | | - | | - | | | - | | - | | - | - | + | | + | + | |

## Claims

1. A *Lactobacillus plantarum* strain deposited in the Spanish Type Culture Collection with number 30089 or mutants derived from same, wherein said mutants have the same or better antimicrobial properties than the strain CECT30089.

2. The strain of the preceding claim, wherein the antimicrobial properties are selected from antifungal properties, antibacterial properties and combinations thereof.

3. A gastro-resistant capsule for use as a probiotic that comprises the strain of bacteria defined in one of claims 1 or 2.

4. The gastro-resistant capsule for use as a probiotic according to the preceding claim, comprising milk from mammals, whey from mammals, plant milk, plant whey and combinations thereof.

5. The gastro-resistant capsule according to the preceding claim, wherein the milk is selected from skimmed milk, semi-skimmed milk, whole milk and combinations thereof.

6. The gastro-resistant capsule for use as a probiotic according to one of claims 3 to 5, comprising at least one additive.

7. The gastro-resistant capsule according to one of claims 3 to 6, wherein the number of bacteria is between 10⁴ and 10¹⁵ cells.

8. An antimicrobial formulation comprising the fermentation product of a substrate, by the strain of claim 1 or 2 from which the bacteria have been separated.

9. The antimicrobial formulation according to the preceding claim, wherein the fermentation substrate is selected from milk from mammals, whey from mammals, plant milk, plant whey and combinations thereof.

10. A method for producing an antimicrobial formulation defined in any of claims 8 to 9, comprising the following steps:
a. fermenting the substrate with a starter culture comprising at least one strain of lactic acid bacteria defined in claim 1 or 2 and
b. at the end of fermentation, separating the lactic acid bacteria from step a) by centrifugation.

11. The method according to the preceding claim, wherein the initial concentration of the starter culture is 10⁴ to 10¹² CFU/ml of the substrate.

12. A method for obtaining a bioactive product, comprising the following steps:
a. preparing a solution of the antimicrobial formulation obtained according to one of claims 10 to 11,
b. dissolving a polymeric matrix in water
c. mixing the product of step a) with the product of step b) to obtain an antimicrobial whey coating formulation and
d. applying the formulation obtained in step c) on a material, to obtain a bioactive product.

13. The method according to the preceding claim, wherein steps a), b) and c) take place simultaneously.

14. The method according to any one of claims 12 to 13, comprising adjusting the pH of the antimicrobial whey coating formulation to:
a. a pH range between 2 and 5, preferably 3, or
b. the same pH of the antimicrobial formulation defined in one of claims 7 or 8.

15. The method according to any one of claims 12 to 14, comprising the addition of at least one additive to the antimicrobial whey coating formulation selected from:
- surfactants, for example, silicone-free hydrophobic solids, polysiloxanes and polysiloxane aqueous solutions, polyether-modified polydimethylsiloxane, paraffin-based oils, polysiloxanes in polyglycol, alkylammonium salts and polymethylalkylsiloxane,
- rheological additives, modified urea solutions, modified hydrophobic polyurethane solution, polyurethanes, aminoplast polyethylene glycol solution, castor oil derivatives, modified urea solutions, polyhydroxycarboxylic acid amide solutions, polyurethane solutions with urea, urea-modified medium-polarity polyamides, polycarboxylic acid ester, and functional oligoamide amine solution, and
- food acids such as acetic acid, lactic acid, malic acid, fumaric acid, citric acid, succinic acid, ascorbic acid, formic acid, sorbic acid, tartaric acid, adipic acid, nicotinic acid and phosphoric acid, preferably phosphoric acid.

16. The method according to any one of claims 12 to 15, comprising applying an adhesion promoter primer solution on the material prior to applying the antimicrobial whey coating formulation.

17. The method according to any one of claims 12 to 16, wherein the material is selected from plastic, preferably PE (polyethylene), polypropylene (OPP), polyethylene terephthalate (PET), low-density polyethylene (LDPE), high-density polyethylene (HDPE), Ethylene-Vinyl-Alcohol (EVOH), paper and ethylene vinyl acetate (EVA), corn starch, polypropylene (PP), polypropylene modified by photoinitiated polymerisation of acrylic acid (PP-g-PAA), polylactic acid (PLA), polyvinyl chloride (PVC), chitosan, fish gelatine, cellulose, paper and combinations thereof.

18. An antimicrobial whey coating formulation that comprises the antimicrobial formulation defined in any one of claims 8 to 9 and a polymeric matrix dissolved in water.

19. The antimicrobial whey coating formulation according to the preceding claim, wherein the polymeric matrix is selected from: polyvinyl alcohol (PVOH), Ethylene-Vinyl-Alcohol (EVOH) copolymers, styrene-acrylic copolymers, acrylic resins, anionic aqueous dispersions, aqueous dispersions, aliphatic polyurethane resins, acrylic-urethane copolymers, polyurethanes, alkyd resins, PVC, polyvinyl acetate, nitrocellulose, polyvinyl butyrate (PVB), polyester (PET), polyester acrylates, urethanes, chitosan, alginates, Hydroxypropyl methylcellulose (HPMC), starch, aminoplast polyethylene glycol solutions, polysaccharides, fatty acids, proteins, waxes, rubbers, collagen, pectins, gelatines and composites.

20. A bioactive product obtained by the method defined in one of claims 12 to 17 that comprises the formulation of one of claims 18 to 19 and a material.

21. The bioactive product according to the preceding claim, which is in the form of a sheet, a film and/or packaging, preferably a film.

22. Use of the bioactive product defined in any of claims 20 to 21 as a microbial growth inhibitor in food products.

23. The use of the bioactive product according to the preceding claim as a fungal growth inhibitor in food products.

24. The use according to one of claims 22 or 23, wherein the food products are dairy products, preferably cheese.

25. Use of the *Lactobacillus plantarum* strain defined in claim 1, as an antimicrobial growth inhibitor in food products.

26. Use of the *Lactobacillus plantarum* strain defined in claim 1 or 2, as a probiotic.
